# EUROPÄISCHE PATENTSCHRIFT

(11) **EP 0 686 698 B1**
(45) Veröffentlichungstag und Bekanntmachung des Hinweises auf die Patenterteilung: **14.03.2001**
(21) Anmeldenummer: 95108740.2
(22) Anmeldetag: 07.06.1995
(51) Int. Cl.: C12P 17/00, C12P 17/10, C12P 17/12, C12R 1/22, C12R 1/38, C12R 1/39, C12R 1/40

(54) **Biotechnologisches Verfahren zur Herstellung von cyclischen S-alpha-Aminocarbonsäuren und R-alpha-Aminocarbonsäureamiden**
Biotechnological process for the production of cyclic S-alpha-aminocarboxylic acids and R-alpha-aminocarboxylic acid amides
Procédé biotechnologique pour la préparation des acides S-alpha-aminocarboxyliques et des amides d'acides R-alpha-aminocarboxyliques

(30) Priorität: 09.06.1994 CH 181394; 13.07.1994 CH 223194
(43) Veröffentlichungstag der Anmeldung: 13.12.1995
(73) Patentinhaber: LONZA AG, CH-3945 Gampel/Wallis (CH)
(72) Erfinder: Kiener, Andreas, Dr. Chem., CH-3930 Visp (Kanton Wallis) (CH); Roduit, Jean-Paul, Dr. Chem., CH-3979 Grône (Kanton Wallis) (CH); Kohr, Jörg, Dr. Chem., CH-3942 Raron (Kanton Wallis) (CH); Shaw, Nicholas, Dr. Biochem., CH-3930 Visp (Kanton Wallis) (CH)
(74) Vertreter: Weinhold, Peter, Dr.rer.nat. Dipl.-Chem.

(56) Entgegenhaltungen:
- EP-A- 0 307 023
- EP-A- 0 348 901
- EP-A- 0 383 403
- EP-A- 0 494 716
- WO-A-89/01525
- GB-A- 1 552 542
- CHEMICAL ABSTRACTS, vol. 110, no. 11, 13.März 1989 Columbus, Ohio, US; abstract no. 93556p, "D-alpha-amino acids and their microbial manufacture" Seite 560; XP002025311 & JP 63 087 998 A (JPN. KOKAI TOKKYO KOHO) 19.April 1988
- CHEMICAL ABSTRACTS, vol. 112, no. 15, 9.April 1990 Columbus, Ohio, US; abstract no. 137533b, "Manufacture of D-alpha-amino acids with Mycobacterium methanolica" Seite 592; XP002025312 & JP 01 215 297 A (JPN. KOKAI TOKKYO KOHO) 29.August 1989

## Beschreibung

Die Erfindung betrifft neue Mikroorganismen, die befähigt sind, α-Aminocarbonsäureamide, in Form des Racemats oder ihrer optisch aktiven Isomere, der allgemeinen Formel 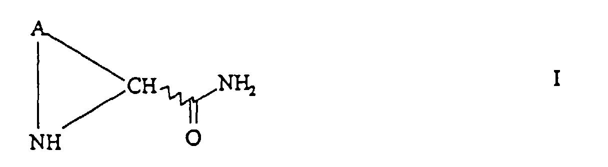 worin A zusammen mit -NH- und -CH- einen gegebenenfalls substituierten 5- oder 6-gliedrigen gesättigten heterocyclischen Ring bedeutet, als einzige Stickstoffquelle zu verwerten und (RS)-α-Aminocarbonsäureamide der bereits definierten Formel I in eine S-α-Aminocarbonsäure der allgemeinen Formel 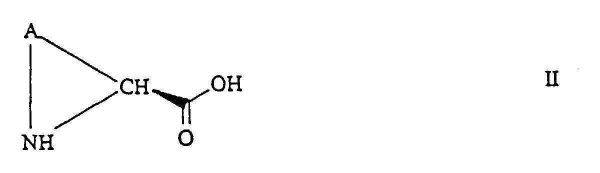 worin A die genannte Bedeutung hat, zu überführen. Diese Mikroorganismen bzw. deren zellfreie Enzyme werden für ein neues Verfahren zur Herstellung von S-α-Aminocarbonsäuren (Formel II) und / oder zur Herstellung von R-α-Aminocarbonsäureamiden der allgemeinen Formel 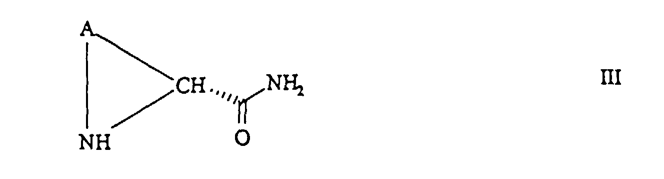 worin A die genannte Bedeutung hat, eingesetzt.

S-α-Aminocarbonsäuren der Formel II wie beispielsweise S-α-Pipecolinsäure sind wichtige Zwischenprodukte zur Herstellung von zahlreichen bioaktiven Verbindungen wie z. B. für Thioridazin oder Pipradol (Ng-Youn-Chen et al., J. Org. Chem., 59 (8), 2075-81, 1994).

Neben zahlreichen chemischen Racematspaltungen von (RS)-Pipecolinsäure bzw. deren Derivaten sind auch biotechnologische Racematspaltungen bekannt. So beschreiben beispielsweise Huh et al., (Biosci., Biotech. Biochem., 56 (12), 2081 - 2082, 1992) die Racematspaltung von (RS)-Pipecolinsäure mittels einer R-Aminosäureoxidase. Dabei wird spezifisch das R-Isomere zu Δ¹-Piperidein-2-carbonsäure oxidiert, wobei S-Pipecolinsäure anfällt. Nach chemischer Reduktion der Δ¹-Piperidein-2-carbonsäure zu (RS)-Pipecolinsäure fällt dann unter dem Einfluss der R-Aminosäureoxidase wieder S-Pipecolinsäure an. Dabei nimmt der Gehalt an R-Pipecolinsäure immer mehr ab. Ein analoges Verfahren zur Herstellung von S-Prolin ist in J. Ferm. Bioeng., 74, 189 - 190, 1992 beschrieben.
Diese beiden Verfahren haben jedoch den Nachteil, dass sie grosstechnisch nicht gangbar sind. Ein weiterer Nachteil besteht darin, dass gereinigte R-Aminosäureoxidase angewendet werden muss.

Desweiteren ist bekannt, dass racemische Pipecolinsäureester unter dem Einfluss einer Lipase aus Aspergillus niger in S-Pipecolinsäure und R-Pipecolinsäureester überführt werden (Ng-Youn-Chen et al., 1994, ibid). Dieses Verfahren hat jedoch den Nachteil, dass S-Pipecolinsäure nur mit einer Enantiomeren-Reinheit von ee = 93% erhalten wird.

Aufgabe der vorliegenden Erfindung ist, ein einfaches und technisch gangbares biotechnologisches Verfahren zur Herstellung von cyclischen S-α-Aminocarbonsäuren zur Verfügung zu stellen, wobei diese in guter Enantiomeren-Reinheit isoliert werden können.

Diese Aufgabe wird mit den Mikroorganismen gemäss Patentanspruch 1 und mit dem Verfahren gemäss Patentanspruch 5 gelöst.

Die erfindungsgemässen Mikroorganismen können aus Bodenproben, Schlamm oder Abwasser unter Zuhilfenahme üblicher mikrobiologischer Techniken isoliert werden. Erfindungsgemäss erfolgt die Isolation dieser Mikroorganismen derart, dass man diese
a) in einem Medium mit einem α-Aminocarbonsäureamid (Formel I) in Form des Racemats oder seiner optisch aktiven Isomere als einzige Stickstoffquelle und mit einer geeigneten Kohlenstoffquelle auf übliche Weise züchtet,
b) aus der durch Züchtung erhaltenen Kultur dann jene selektioniert, die stabil sind und befähigt sind -α-Aminocarbonsäureamid (Formel I) in eine S-α-Aminocarbonsäure (Formel II) zu überführen.

Demzufolge können alle Mikroorganismen angewendet werden, die spezifisch diese S-Aminosäure-Amidasen enthalten. Zweckmässig werden die Mikroorganismen selektioniert, die Piperazincarboxamid oder Pipecolinamid, in Form des Racemats oder der optisch aktiven Isomeren, als einzige Stickstoffquelle verwerten. Vorzugsweise werden die selektioniert, welche S-Piperazincarboxamid oder S-Pipecolinamid als einzige Stickstoffquelle verwerten.

Als Kohlenstoffquelle können die Mikroorganismen beispielsweise Zucker, Zuckeralkohole, Carbonsäuren oder Alkohole als Wachstumssubstrat nützen. Als Zucker können Hexosen wie beispielsweise Glucose oder Pentosen angewendet werden. Als Carbonsäuren können Di- oder Tricarbonsäuren bzw. deren Salze verwendet werden wie beispielsweise Citronensäure oder Succinat. Als Alkohol kann ein dreiwertiger Alkohol Verwendung finden wie beispielsweise Glycerin. Vorzugsweise wird als Kohlenstoffquelle ein dreiwertiger Alkohol wie Glycerin eingesetzt.

Als Selektions- und Anzuchtmedium können die in der Fachwelt üblichen verwendet werden, wie beispielsweise das Mineralsalzmedium gemäss Kulla et al., (Arch. Microbiol., 135, 1 - 7, 1983) oder das in Tabelle 1 beschriebene. Vorzugsweise wird das in Tabelle 1 beschriebene angewendet.

Während der Anzucht und Selektion werden zweckmässig die wirksamen Enzyme der Mikroorganismen induziert. Als Enzym-Induktor kann beispielsweise Piperazincarboxamid, Pipecolinamid oder Acetamid angewendet werden.

Zweckmässig erfolgt die Anzucht und Selektion bei einer Temperatur von 15 bis 50 °C, vorzugsweise von 20 bis 45 °C und bei einem pH-Wert zwischen pH 5 und pH 10, vorzugsweise zwischen pH 6 und pH 9.

Bevorzugte Mikroorganismen mit spezifischer S-Aminosäure-Amidase-Aktivität sind Piperazincarboxamid-verwertende Mikroorganismen der Gattung Klebsiella, insbesondere der Spezies Klebsiella pneumoniae mit der Bezeichnung DSM 9175 und DSM 9176 oder der Spezies Klebsiella terrigena mit der Bezeichnung DSM 9174, sowie deren funktionell aequivalente Varianten und Mutanten. Diese Mikroorganismen wurden am 25.04.1994 bei der Deutschen Sammlung von Mikroorganismen und Zellkulturen GmbH, Mascheroderweg 1b, D-38124 Braunschweig, gemäss Budapester Vertrag hinterlegt.
Weitere geeignete Mikroorganismen sind Pipecolinamid-verwertende Mikroorganismen der Gattung Pseudomonas, insbesondere der Spezies Pseudomonas putida mit der Bezeichnung DSM 9923 oder der Spezies Pseudomonas fluorescens mit der Bezeichnung DSM 9924, sowie deren funktionell aequivalente Varianten und Mutanten. Diese Mikroorganismen wurden am 20.04.1995 bei der Deutschen Sammlung von Mikroorganismen und Zellkulturen GmbH, Mascheroderweg 1b, D-38124 Braunschweig, gemäss Budapester Vertrag hinterlegt.

Unter "funktionell aequivalente Varianten und Mutanten" werden Mikroorganismen verstanden, die im wesentlichen dieselben Eigenschaften und Funktionen wie die Ursprungsmikroorganismen besitzen. Derartige Varianten und Mutanten können zufällig, z. B. durch UV-Bestrahlung,gebildet werden.

Wissenschaftliche Beschreibung von Mikroorganismus DSM 9175 identifiziert als Klebsiella pneumoniae

| Eigenschaften des Stammes | | |
|---|---|---|
| Zellform | Stäbchen | |
| Breite µm | 0,8 - 1,0 | |
| Länge µm | 1,0 - 3,0 | |
| | | |
| Beweglichkeit | - | |
| | | |
| Gram-Reaktion | - | |
| Lyse durch 3% KOH | + | |
| Aminopeptidase (Cerny) | + | |
| | | |
| Sporen | - | |
| | | |
| Oxidase | - | |
| | | |
| Catalase | + | |
| | | |

| Wachstum | | |
|---|---|---|
| anaerob | + | |
| | | |

| Säure aus (OF-Test) | | |
|---|---|---|
| Glucose aerob | + | |
| Glucose anaerob | + | |
| | | |
| Gas aus Glucose | + | |
| | | |

| Säure aus (ASS) | | |
|---|---|---|
| Glucose | + | |
| Fructose | + | |
| Xylose | + | |
| Erythrit | - | |
| Adonit | + | |
| D-Mannose | + | |
| L-Rhamnose | + | |
| Dulcit | - | |
| Inosit | + | |
| Sorbit | + | |
| α-Methyl-D-glucosid | + | |
| Cellobiose | + | |
| Maltose | + | |
| Lactose | + | |
| L-Sorbose | + | |
| L-Fucose | - | |
| D-Arabitol | + | |
| | | |
| ONPG | + | |
| ADH | - | |
| | | |
| LDC | + | |
| | | |
| ODC | - | |
| | | |
| VP | + | |
| | | |
| Indol | - | |
| | | |
| H₂S-Bildung | - | |
| | | |
| Simmons Citrat | + | |
| | | |
| Phenylalanindesaminase | - | **Abkürzungen:** |
| | | |
| Urease | - | ASS = Acetylsalicylsäure |
| | | OF = Oxidation-Fermentation |
| Hydrolyse von | | ONPG = O-Nitrophenylgalactosidase |
| Gelatine | - | ADH = Alkoholdehydrogenase |
| DNA | - | VP = Voges Proskauer |

Wissenschaftliche Beschreibung von Mikroorganismus DSM 9176 identifiziert als Klebsiella pneumoniae

| Eigenschaften des Stammes | |
|---|---|
| Zellform | Stäbchen |
| Breite µm | 0,8 - 1,0 |
| Länge µm | 1,0 - 3,0 |
| | |
| Beweglichkeit | - |
| | |
| Gram-Reaktion | - |
| Lyse durch 3% KOH | + |
| Aminopeptidase (Cerny) | + |
| | |
| Sporen | - |
| | |
| Oxidase | - |
| | |
| Catalase | + |
| | |

| Wachstum | |
|---|---|
| anaerob | + |
| | |

| Säure aus (OF-Test) | |
|---|---|
| Glucose aerob | + |
| Glucose anaerob | + |
| | |
| Gas aus Glucose | + |
| | |

| Säure aus (ASS) | |
|---|---|
| Glucose | + |
| Fructose | + |
| Xylose | + |
| Erythrit | - |
| Adonit | + |
| D-Mannose | + |
| L-Rhamnose | + |
| Dulcit | - |
| Inosit | + |
| Sorbit | + |
| α-Methyl-D-glucosid | + |
| Cellobiose | + |
| Maltose | + |
| Lactose | + |
| L-Sorbose | - |
| L-Fucose | + |
| D-Arabitol | + |
| | |
| ONPG | + |
| | |
| ADH | - |
| | |
| LDC | + |
| | |
| ODC | - |
| | |
| VP | + |
| | |
| Indol | - |
| | |
| H₂S-Bildung | - |
| | |
| Simmons Citrat | + |
| | |
| Phenylalanindesaminase | - |
| | |
| Urease | - |
| | |
| Hydrolyse von | |
| Gelatine | - |
| DNA | - |

Wissenschaftliche Beschreibung von Mikroorganismus DSM 9174 identifiziert als Klebsiella terrigena

| Eigenschaften des Stammes | |
|---|---|
| Zellform | Stäbchen |
| Breite µm | 0,8 - 1,0 |
| Länge um | 1,0 - 2,0 |
| | |
| Beweglichkeit | - |
| | |
| Gram-Reaktion | - |
| Lyse durch 3% KOH | + |
| Aminopeptidase (Cerny) | + |
| | |
| Sporen | - |
| | |
| Oxidase | - |
| | |
| Catalase | + |
| | |

| Wachstum | |
|---|---|
| anaerob | + |
| | |

| Säure aus (OF-Test) | |
|---|---|
| Glucose aerob | + |
| Glucose anaerob | + |
| | |
| Gas aus Glucose | + |
| | |

| Säure aus (ASS) | |
|---|---|
| Glucose | + |
| Fructose | + |
| Xylose | + |
| Erythrit | - |
| Adonit | + |
| D-Mannose | + |
| L-Rhamnose | + |
| Dulcit | - |
| Inosit | + |
| Sorbit | + |
| α-Methyl-D-glucosid | + |
| Cellobiose | + |
| Maltose | + |
| Lactose | + |
| L-Sorbose | + |
| L-Fucose | + |
| D-Arabitol | + |
| 5-Ketogluconat | + |
| | |
| ONPG | + |
| | |
| ADH | - |
| | |
| LDC | + |
| | |
| ODC | + |
| | |
| VP | + |
| | |
| Indol | - |
| | |
| H₂S-Bildung | - |
| | |
| Simmons Citrat | + |
| | |
| Phenylalanindesaminase | - |
| | |
| Urease | - |
| | |
| Hydrolyse von | |
| Gelatine | - |
| DNA | - |

Das erfindungsgemässe Verfahren zur Herstellung von S-α-Aminocarbonsäuren der allgemeinen Formel 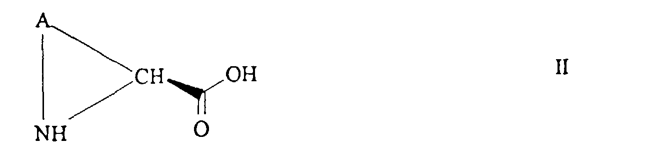 worin A die genannte Bedeutung hat, und / oder von R-α-Aminocarbonsäureamiden der allgemeinen Formel 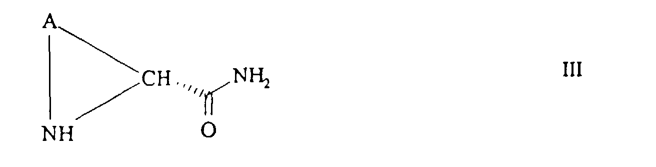 worin A die genannte Bedeutung hat, erfolgt derart, dass man im (RS)-α-Aminocarbonsäureamid der allgemeinen Formel 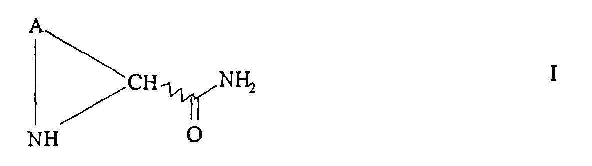 worin A die genannte Bedeutung hat, das S-α-Aminocarbonsäureamid mittels den spezifischen bereits beschriebenen Mikroorganismen oder mittels zellfreien Enzymen aus diesen Mikroorganismen zur S-α-Aminocarbonsäure überführt und isoliert, wobei bei der Biotransformation neben der S-α-Aminocarbonsäure das R-α-Aminocarbonsäureamid anfällt, welches gegebenenfalls isoliert wird.

Die Edukte, die (RS)-α-Aminocarbonsäureamide der allgemeinen Formel 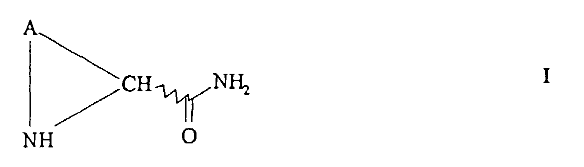 worin A zusammen mit -NH- und -CH- einen 5- oder 6-gliedrigen gesättigten heterocyclischen Ring, gegebenenfalls substituiert, bedeutet, können aus den entsprechenden aromatischen Amiden durch fachmännisch übliche Hydrierung gewonnen werden.

Als Aminocarbonsäureamide der Formel I mit einem 5-gliedrigen gesättigten heterocyclischen Ring können gegebenenfalls substituiertes Prolinamid, Pyrazolidincarboxamid, Imidazolidincarboxamid, Oxazolidincarboxamid, Isoxazolidincarboxamid, Thiazolidincarboxamid oder Triazolidincarboxamid angewendet werden. Als substituiertes Prolinamid kann beispielsweise Indolinamid verwendet werden.

Als Aminocarbonsäureamide der Formel I mit einem 6-gliedrigen gesättigten heterocyclischen Ring können Piperazincarboxamid, Pipecolinamid, Morpholincarboxamid, Perhydrochinolincarboxamid (Chinolinancarboxamid), Perhydroisochinolincarboxamid (Isochinolinancarboxamid), Perhydrochinoxalincarboxamid (Chinoxalinancarboxamid), die ebenfalls gegebenenfalls substituiert sind, angewendet werden. Vertreter von Aminocarbonsäureamiden mit einem substituierten 6-gliedrigen gesättigten heterocyclischen Ring können sein C₁-C₄-Alkyl-substituierte H₂N-CH₂-substituierte wie z. B. 4-Methylpipecolinamid, wie beispielsweise 4-Aminomethylpipecolinamid oder CN-substituierte wie 4-Cyanpipecolinamid. Vorzugsweise wird Piperazincarboxamid, Pipecolinamid oder 4-Methylpipecolinamid verwendet.

Die Enzyme für das zellfreie System können durch fachmännisch übliches Aufschliessen der Mikroorganismen gewonnen werden. Hierzu kann beispielsweise die Ultraschall-, French-Press oder Lysozym-Methode verwendet werden. Diese zellfreien Enzyme können auch auf einem geeigneten Trägermaterial immobilisiert werden.

Für das Verfahren besonders geeignet, sind die zuvor beschriebenen spezifischen Mikroorganismen der Spezies Klebsiella terrigena DSM 9174, Klebsiella pneumoniae DSM 9175 und DSM 9176, der Spezies Pseudomonas putida DSM 9923 und Pseudomonas fluorescens DSM 9924 sowie deren zellfreie Enzyme. Insbesondere wird das Verfahren mit den Mikroorganismen Klebsiella terrigena DSM 9174, Klebsiella pneumoniae DSM 9175 und DSM 9176 durchgeführt. Ebenfalls geeignet sind die funktionell aequivalenten Varianten und Mutanten der beschriebenen Mikroorganismen.

Die Biotransformation kann nach üblichem Anzüchten der Mikroorganismen mit ruhenden Zellen (nicht wachsende Zellen, die keine Kohlenstoff- und Energiequelle mehr benötigen) oder mit wachsenden Zellen durchgeführt werden.

Als Medium für das Verfahren mit ruhenden Zellen können die fachmännisch üblichen dienen, wie beispielsweise das zuvor beschriebene Mineralsalzmedium gemäss Kulla et al., 1983 (ibid), niedermolare Phosphatpuffer, HEPES-Puffer, oder das in Tabelle 1 beschriebene Medium. Für das Verfahren mit wachsenden Zellen wird üblicherweise ein Medium enthaltend eine Kohlenstoff- und Stickstoffquelle wie beispielsweise handelsübliche Medien oder das Medium gemäss Tabelle 1 angewendet. Vorzugsweise wird das Verfahren in dem Medium gemäss Tabelle 1 durchgeführt.

Zweckmässig wird die Biotransformation unter einmaliger oder kontinuierlicher Zugabe von (RS)-α-Aminocarbonsäureamid so durchgeführt, dass die Konzentration an (RS)-α-Aminocarbonsäureamid 20 Gew.%, vorzugsweise 10 Gew.%, nicht übersteigt.

Der pH-Wert des Mediums kann in einem Bereich von pH 5 bis pH 11, vorzugsweise von pH 7 bis pH 10, liegen.

Zweckmässig wird die Biotransformation bei einer Temperatur von 25 bis 65 °C, vorzugsweise von 30 bis 60 °C durchgeführt.

Nach einer üblichen Umsetzungszeit von 1 bis 100 h ist das S-α-Aminocarbonsäureamid der Formel I vollständig zur S-α-Aminocarbonsäure umgewandelt, wobei auch R-a-Aminocarbonsäureamid anfällt.

Die auf diese Weise gewonnene S-α-Aminocarbonsäure und / oder das R-α-Aminocarbonsäureamid können durch übliche Aufarbeitungsmethoden wie z. B. durch Ansäuern, Chromatographie oder Extraktion isoliert werden.

### Beispiele:

### Beispiel 1

### a) Isolation von Mikroorganismen, die befähigt sind racemisches Piperazincarboxamid als einzige Stickstoffquelle zu verwerten:

Für die Isolation von Mikroorganismen, die befähigt sind, racemisches Piperazincarboxamid als einzige Stickstoffquelle zu verwerten, wurde das A-Medium, dessen Zusammensetzung in Tabelle 1 gegeben ist, verwendet. 100 ml dieses Mediums wurden in einen 300 ml Erlenmeyerkolben gegeben und mit verschiedenen Erdproben (2 g) aus dem Werksareal der LONZA AG in Visp, Schweiz versetzt. Die Kolben wurden ruhend bei 30 °C während 5 Tagen inkubiert. Dann wurde 1 ml dieses A-Mediums verwendet, um einen frischen Kolben mit dem gleichen Medium zu beimpfen. Dieser Kolben wurde wiederum unter den gleichen Bedingungen inkubiert. Insgesamt wurde dieser Anreicherungszyklus 5-mal wiederholt. Danach wurden die Anreicherungen auf Agar-Medium (A-Medium mit zusätzlich 16 gl⁻¹ Agar) zu Einzelkolonien ausgestrichen.
Die isolierten Mikroorganismen wurden im folgenden qualitativen Testsystem auf stereoselektive Amidasen hin untersucht. Einzelkolonien wurden verwendet, um 100 ml A-Medium in 300 ml Erlenmeyerkolben zu beimpfen. Diese Kolben wurden drei Tage auf einer Schüttelmaschine bei 30 °C inkubiert, wobei die Kulturen bereits nach einem Tag ausgewachsen waren. Danach wurden die zellfreien Kulturüberstände mittels Dünnschichtchromatographie (Kieselgel, Laufmittel: 11 Teile Ethanol, 6 Teile CHCl₃, 6 Teile NH₄OH (25%), Nachweis mittels Ninhydrin) auf den Gehalt an Piperazincarbonsäure und Piperazincarboxamid hin untersucht. Mikroorganismen, die etwa die Hälfte der eingesetzten Menge (RS)-Piperazincarboxamid umgesetzt hatten, wurden für biochemische Untersuchungen verwendet, um Festzustellen, welche Stämme S-spezifische Amidasen enthielten.

### b) Biochemische Untersuchungen zur Identifizierung von Mikroorganismen mit S-spezifischen Amidasen:

Zur Herstellung von Protein-Rohextrakt wurden die Zellen in 11 A-Medium bei 30 °C angezogen und anschliessend geerntet und gewaschen. 5 g Zellen (Nassgewicht) wurden in 10 ml 69 mMol Phosphatpuffer, pH 7,0, resuspendiert und mittels einer FRENCH®-Presse aufgeschlossen. Der Rohextrakt wurde nach einer Zentrifugation von 2 h bei 40'000 x g in Portionen bei -20 °C eingefroren. Zur Bestimmung der Stereoselektivität wurden die Hydrolyseraten von R-Prolinamid und S-Prolinamid verglichen. Dazu wurde folgender Enzymtest verwendet: Assayvolumen 1 ml, enthaltend 69 mMol Phosphatpuffer, pH 7,0, 100 - 800 µg Protein-Rohextrakt, 2 mg S- resp. R-Prolinamid·HCl, Inkubationszeit 1 - 24 h, Inkubationstemperatur 30 °C, Detektion mit Ninhydrin nach Dünnschichtchromatographie (siehe oben). Die Amidasen der Stämme DSM 9174, DSM 9175 und DSM 9176 zeigten eine sehr langsame Hydrolyse von R-Prolinamid. Diese Stämme wurden für die Herstellung optisch aktiver zyklischer α-Aminosäurederivate verwendet.

Die Rohextrakte der Stämme DSM 9175 und DSM 9176 zeigten unter den gleichen Bedingungen eine Hydrolyse von (RS)-Piperazincarboxamid und (RS)-Pipecolinamid. Durch Veränderung der Inkubationstemperatur und des pH-Wertes der Assay-Lösung wurde festgestellt, dass die spezifische Aktivität der Amidasen zwischen einer Temperatur von
30 - 60 °C und einem pH-Wert von 7 - 10 am grössten war.

**Tabelle 1**

| | |
|---|---|
| **A-Medium:** Für dieses Medium wurde das unten beschriebene Minimal-Medium mit zusätzlich 2 gl⁻¹ (RS)-Piperazincarboxamid und 10 gl⁻¹ Glycerin versetzt. | |
| **B-Medium:** Für dieses Medium wurde das unten beschriebene Minimal-Medium mit zusätzlich 1 gl⁻¹ (RS)-Pipecolinamid und 4 gl⁻¹ Glucose versetzt. | |

| **Minimal-Medium:** | |
|---|---|
| Zusammensetzung | Konzentration (mg/l) |
| | |
| Hefeextrakt | 500 |
| Na₂SO₄ | 100 |
| Na₂HPO₄ | 2000 |
| KH₂PO₄ | 1000 |
| NaCl | 3000 |
| MgCl₂×6H₂O | 400 |
| CaCl₂×2H₂O | 14,5 |
| FeCl₃×6H₂O | 0,8 |
| ZnSO₄×7H₂O | 100×10⁻³ |
| MnCl₂×4H₂O | 90×10⁻³ |
| H₃BO₃ | 300×10⁻³ |
| CoCl₂×6H₂O | 200×10⁻³ |
| CuCl₂×2H₂O | 10×10⁻³ |
| NiCl₂×6H₂O | 20×10⁻³ |
| NaMoO₄×2H₂O | 30×10⁻³ |
| EDTA Na₂×2H₂O | 5 |
| FeSO₄×7H₂O | 2 |

### Beispiele 2 - 4

### Herstellung von S-Piperazincarbonsäure:

Für die Herstellung von S-Piperazincarbonsäure mit den Stämmen DSM 9174, DSM 9175 und DSM 9176 wurden folgende Bedingungen gewählt. Ein 1,5 l Fermenter, ausgerüstet mit einer pH-Steuerung, mit 1 l Arbeitsvolumen wurde für die Biotransformationen verwendet. Für Fermentationen wurde im A-Medium die Menge Glycerin auf 30 gl⁻¹ und die Menge (RS)-Piperazincarboxamid auf 20 gl⁻¹ erhöht. Die Zellen wurden bei pH 7,0, einer Temperatur von 30 °C und einer Belüftungsrate von 0,5 lmin⁻¹ angezogen. In einem Fall (Beispiel 4) wurden die Zellen zuerst 16 Stunden unter diesen Bedingungen angezogen, dann wurde die Temperatur auf 40 °C und der pH des Mediums auf 8,0 erhöht. Nach bestimmten Zeiten wurde mittels Dünnschichtchromatographie die Menge gebildeter S-Piperazincarbonsäure abgeschätzt und die Fermentationen wurden nach 36 - 72 h abgebrochen, sobald ungefähr die Hälfte des eingesetzten Piperazincarboxamids umgesetzt war. Zu diesem Zeitpunkt waren die optischen Dichten der Zellsuspension bei 650 nm zwischen 6 und 10. Zur Isolierung von S-Piperazincarbonsäure wurde die zellfreie Lösung unter reduziertem Druck auf 100 ml aufkonzentriert.

Mit konzentrierter HCl wurde die Lösung auf pH 1,0 angesäuert, um die Säure als Dihydrochlorid auszufällen. Die isolierte Säure wurde in 0,1 M HCI umkristallisiert und getrocknet. Zur Bestimmung des ee-Wertes (enantiomeric excess) der gebildeten Säure wurde die Säure zuerst mit 2,3,4,6-Tetra-O-acetyl-β-D-glucopyranosylisothiocyanat derivatisiert und mittels Kapillar-Elektrophorese analysiert (siehe Tabelle 2 für Bedingungen der Kapillar-Elektrophorese). Die Ergebnisse sind in Tabelle 3 beschrieben.

**Tabelle 2**

| Kapillar-Elekrophorese Bedingungen | |
|---|---|
| CE Apparatur | Hewlett-Packard HP ^{3D}CE |
| Detektor | Hewlett-Packard Diode-Array-Detektor |
| Puffer | 10 mM Di-Natriumhydrogenphosphat, 10 mM Borsäure, 150 mM Natriumdodecylsulfat, pH 9,0 |
| Elektrolyt | 900 ml Puffer plus 100 ml Methanol |
| Kapillare | HP G1600-61211 |
| Elektrisches Feld | 20 kV |
| Strom | ca. 24 - 30 µA |
| Ofentemperatur | 20 °C |
| Detektoreinstellung | 210 nm (Bandbreite 5 nm) |
| Migrationszeit | ca. 17,1 min (S-Säure) |
| | ca. 17,7 min (R-Säure) |

**Tabelle 3**

| **Beispiel Stamm** | **Rohprodukt** | **Umkristallisiert** | **Ausbeute** | **ee-Wert** |
|---|---|---|---|---|
| Nr. 2 DSM 9175 | 12,82 g | 10,74 g | 68,3 % | 99,6 |
| Nr. 3 DSM 9174 | 15,53 g | 13,1 g | 83,3 % | 99,4 |
| Nr. 4 DSM 9176 | 21,23 g | 11,32 g | 72,0 % | 99,6 |

### Beispiel 5

### Herstellung von S-Pipecolinsäure mittels Klebsiella:

Ruhende Zellen der Stämme Klebsiella pneumoniae DSM 9175 und DSM 9176 setzten 20 gl⁻¹ (RS)-Pipecolinamid bei 47 °C und einem pH-Wert von 8,0 innerhalb von 6 h zur (S)-Säure um.

Wurde diese Umsetzung mit Klebsiella pneumoniae DSM 9175 durchgeführt wurde S-Pipecolinsäure mit einem ee-Wert von 96,5% erhalten

### Beispiel 6

### Herstellung von S-4-Methylpipecolinsäure

Für diese Umsetzung wurde racemisches 4-Methylpipecolinamid (Substrat) mittels den Protein-Rohextrakten von Klebsiella pneumoniae DSM 9175 oder Klebsiella terrigena DSM 9174 analog zu Beispiel 1b umgesetzt. Nach 24 h Inkubation, bei pH 8,0 und 47 °C, waren von einer 0,2%igen Substratlösung ca. 50% des eingesetzten Amids (gemessen anhand von TLC-Analytik) umgesetzt.

### Beispiel 7

### Isolation von Mikroorganismen, die Pipecolinamid als einzige Stickstoffquelle verwerten:

100 ml B-Medium wurden in 300 ml Erlenmeyerkolben gegeben und mit ca. 2 g Erdproben versetzt. Die Kolben wurden während 3 Tagen bei Raumtemperatur ruhend inkubiert. Anschliessend wurden frische Kolben mit demselben Medium mit 2 ml aus den vorigen Kulturen versetzt und ruhend während 4 Tagen bei 30 °C inkubiert. Dieser Anreicherungszyklus wurde insgesamt 3 mal wiederholt, wobei die zwei letzten Schritte steril durchgeführt wurden. Der letzte Schritt wurde noch zusätzlich auf einer Schüttelmaschine bei 140 rpm durchgeführt wurde. Anschliessend wurden die Anreicherungen auf B-Medium mit zusätzlich 16 g/l Agar zu Einzelkolonien ausgestrichen. Es wurden dann jene Kolonien selektioniert, deren Eigenschaft (RS)-Pipecolinamid zu Pipecolinsäure umzusetzen, stabil war. Dafür wurden die Kulturen auf Nutrient Agar ausgestrichen und nach einigen Tagen Wachstum, wieder von der Nutrient-Agar Platte auf Pipecolinamid-Glucose Platten ausgestrichen. Die 2 Stämme, die die erwünschte stabile Eigenschaft zeigten, wurden nach Gram-Färbung und Oxidase-Test mit 20 NE API-Streifen identifiziert. Es wurden 2 Pseudomonas Arten identifiziert: Pseudomonas putida, DSM 9923 und, Pseudomonas fluorescens, DSM 9924.

### Beispiel 8

### Herstellung von S-Pipecolinsäure:

Mit den aus Beispiel 7 isolierten Stämmen wurden 1% Pipecolinamid-Biotransformationen, (Substratkonzentration 1%) in 0,1 M Phosphatpuffer bei pH 7,0, 30 °C und 130 rpm durchgeführt. Die Biomasse aus den Vorkulturen wurde aufkonzentriert und in 0,1 M Phosphatpuffer pH 7,0 aufgenommen, um eine Biomassekonzentration von OD₆₅₀ₙₘ = 10 zu erhalten. Die Ansätze wurden auf einer Schüttelmaschine bei 30 °C, 130 rpm inkubiert und Proben zu verschiedenen Zeitpunkten gezogen. Die zellfreien Überstände wurden mittels Dünnschichtchromatographie (Kieselgel, Laufmittel: 11 Teile Ethanol, 6 Teile CHCl₃, 6 Teile NH₄OH 25%) auf den Gehalt an restlichem Amid und gebildeter Pipecolinsäure hin untersucht. Anschliessend wurden die zellfreien Überstände einer HPLC-Untersuchung (Isothiocyanat-Derivatisierung) unterworfen, um die Enantiomeren-Reinheit der gebildeten Pipecolinsäure zu prüfen. Die Ergebnisse zeigten, dass Pseudomonas putida und Pseudomonas fluorescens S-Pipecolinsäure mit einer optischen-Reinheit von über 90% produzierten. Die optimalen Bedingungen für die Biotransformation mit Pseudomonas putida waren bei pH 8,0 und einer Temperatur von 30 °C, für Pseudomonas fluorescens pH 8,0 und 50 °C.
Wurde die Biotransformation mit Pseudomonas putida durchgeführt, erhielt man S-Pipecolinsäure mit einem ee-Wert von 95,0%; wurde die Biotransformation mit Pseudomonas fluorescens durchgeführt, erhielt man S-Pipecolinsäure mit einem ee-Wert von 97,3%.

### Beispiel 9

### Herstellung von S-Piperazincarbonsäure:

Es wurde mit den 2 isolierten Stämmen, wie in Beispiel 7 für Pipecolinamid beschrieben, eine 1% Piperazincarboxamid-Biotransformation bei pH 7,0, 30 °C und 130 rpm durchgeführt. Die zellfreien Überstände wurden ebenso auf den Gehalt an restlichem Piperazincarboxamid und gebildeter Piperazincarbonsäure mit Dünnschichtchromatographie hin untersucht. Eine HPLC-Analyse ermöglichte die optische Reinheit der gebildeten Piperazincarbonsäure zu prüfen. Wenn die Biotransformation mit Pseudomonas putida durchgeführt würde, erhielt man S-Piperazincarbonsäure mit einem ee-Wert von 73,9%; wenn die Biotransformation mit Pseudomonas fluorescens durchgeführt würde, erhielt man S-Piperazincarbonsäure mit einem ee-Wert von 59,5%.

## Patentansprüche

1. Mikroorganismen, dadurch gekennzeichnet, dass sie befähigt sind, α-Aminocarbonsäureamide, in Form des Racemats oder ihrer optisch aktiven Isomere, der allgemeinen Formel 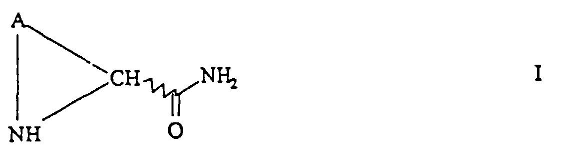 worin A zusammen mit -NH und -CH einen gegebenenfalls substituierten 5- oder 6-gliedrigen gesättigten heterocyclischen Ring bedeutet, als einzige Stickstoffquelle zu verwerten und (RS)-α-Aminocarbonsäureamide der bereits definierten Formel I in eine S-α-Aminocarbonsäure der allgemeinen Formel 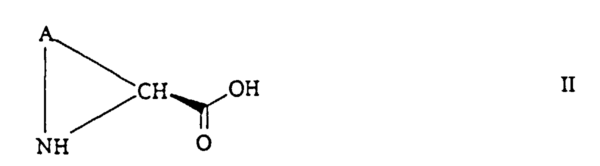 worin A die genannte Bedeutung hat, zu überführen.

2. Mikroorganismen nach Anspruch 1, die befähigt sind, Pipecolinamid oder Piperazincarboxamid in Form des Racemats oder seiner optisch aktiven Isomere als einzige Stickstoffquelle zu verwerten.

3. Mikroorganismen nach Anspruch 2 der Gattung Klebsiella oder Pseudomonas.

4. Mikroorganismen nach Anspruch 3 der Spezies Pseudomonas putida DSM 9923, der Spezies Pseudomonas fluorescens DSM 9924, der Spezies Klebsiella terrigena DSM 9174, der Spezies Klebsiella pneumoniae DSM 9175 und DSM 9176, sowie deren fünktionell aequivalente Varianten und Mutanten.

5. Verfahren zur Herstellung von S-α-Aminocarbonsäuren der allgemeinen Formel 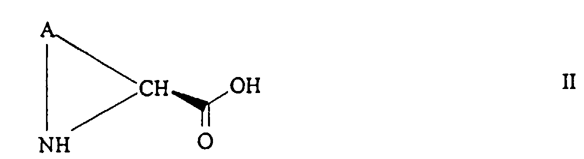 worin A die in Anspruch 1 genannte Bedeutung hat und / oder von R-α-Aminocarbonsäureamiden der allgemeinen Formel 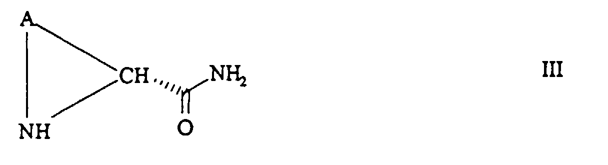 worin A die genannte Bedeutung hat, dadurch gekennzeichnet, dass man im (RS)-α-Aminocarbonsäureamid der allgemeinen Formel 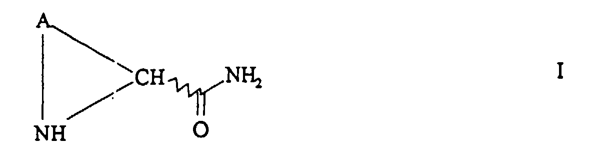 worin A die genannte Bedeutung hat, das S-α-Aminocarbonsäureamid mittels den Mikroorganismen gemäss Anspruch 1 oder mittels zellfreien Enzymen aus diesen Mikroorganismen zur S-α-Aminocarbonsäure überführt und isoliert, wobei bei der Biotransformation neben der S-α-Aminocarbonsäure das R-α-Aminocarbonsäureamid anfällt, das gegebenenfalls isoliert wird.

6. Verfahren nach Patentanspruch 5, dadurch gekennzeichnet, dass man die Biotransformation mittels Mikroorganismen der Spezies Klebsiella terrigena DSM 9174, oder Klebsiella pneumoniae DSM 9175 und DSM 9176 oder mit deren funktionell aequivalente Varianten und Mutanten oder mit zellfreien Enzymen aus diesen Mikroorganismen durchführt.

7. Verfahren nach Patentanspruch 5 oder 6, dadurch gekennzeichnet, dass man als (RS)-α-Aminocarbonsäureamid (RS)-Pipecolinamid, (RS)-Piperazincarboxamid, (RS)-4-Methylpipecolinamid oder (RS)-Prolinamid, verwendet.

8. Verfahren nach mindestens einem der Patentansprüche 5 bis 7, dadurch gekennzeichnet, dass man die Biotransformation unter einmaliger oder kontinuierlicher Zugabe des (RS)-α-Aminocarbonsäureamids durchführt, so dass die Konzentration an (RS)-α-Aminocarbonsäureamid im Kulturmedium 20 Gew.% nicht übersteigt.

9. Verfahren nach mindestens einem der Patentansprüche 5 bis 8, dadurch gekennzeichnet, dass man die Biotransformation bei einem pH-Wert von 7 bis 10 und bei einer Temperatur von 30 bis 60 °C durchführt.

## Claims

1. Microorganisms, characterised in that they are capable of utilising α-aminocarboxamides, in the form of the racemate or of their optically active isomers, corresponding to the general formula 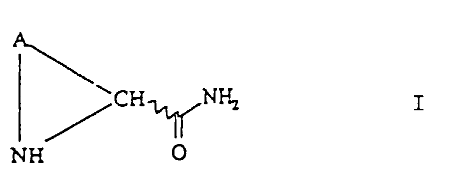 wherein A together with -NH and -CH denote an optionally substituted 5- or 6-membered saturated heterocyclic ring, as the sole nitrogen source and of converting (RS)-α-aminocarboxamides corresponding to the already defined formula I into an S-α-amino acid corresponding to the general formula 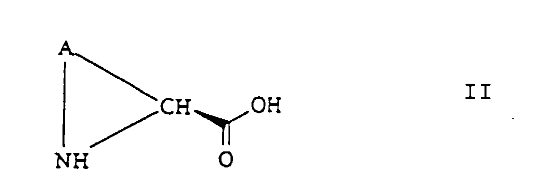 wherein A has the meaning given above.

2. Microorganisms according to claim 1, which are capable of utilising pipecoline amide or piperazine carboxamide, in the form of the racemate or of its optically active isomers, as the sole nitrogen source.

3. Microorganisms according to claim 2, of the genus Klebsiella or Pseudomonas.

4. Microorganisms according to claim 3, of the species Pseudomonas putida DSM 9923, of the species Pseudomonas fluorescens DSM 9924, of the species Klebsiella terrigena DSM 9174, of the species Klebsiella pneumoniae DSM 9175 and DSM 9176, as well as their functionally equivalent variants and mutants.

5. Process for the production of S-α-amino acids corresponding to the general formula 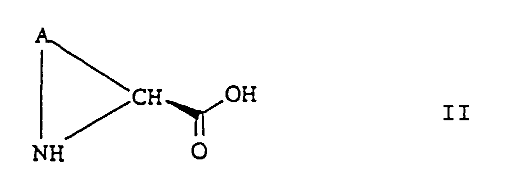 wherein A has the meaning given in claim 1 and/or of R-α-aminocarboxamides corresponding to the general formula 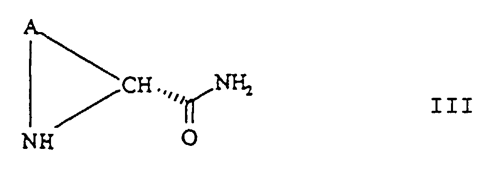 wherein A has the meaning given above, characterised in that, in the (RS)-α-aminocarboxamide corresponding to the general formula 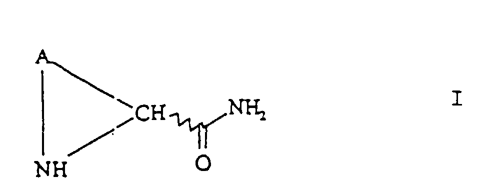 wherein A has the meaning given above, the S-α-aminocarboxamide is converted to the S-α-amino acid by means of the microorganisms according to claim 1 or by means of cell-free enzymes from these microorganisms and is isolated, with the R-α-aminocarboxamide being obtained in addition to the S-α-amino acid during the biotransformation and optionally being isolated.

6. Process according to claim 5, characterised in that the biotransformation is carried out by means of microorganisms of the species Klebsiella terrigena DSM 9174, or Klebsiella pneumoniae DSM 9175 and DSM 9176 or by their functionally equivalent variants and mutants or by cell-free enzymes from these microorganisms.

7. Process according to claim 5 or 6, characterised in that (RS)-pipecoline amide, (RS)-piperazine carboxamide, (RS)-4-methylpipecoline amide or (RS)-prolinamide are used as the (RS)-α-aminocarboxamide.

8. Process according to at least one of claims 5 to 7, characterised in that the biotransformation is carried out with single or continuous addition of the (RS)-α-aminocarboxamide, in such a way that the concentration of (RS)-α-aminocarboxamide in the culture medium does not exceed 20 wt.%.

9. Process according to at least one of claims 5 to 8, characterised in that the biotransformation is carried out at a pH value of 7 to 10 and at a temperature of 30°C to 60°C.

## Revendications

1. Microorganismes caractérisés en ce qu'ils sont capables d'utiliser comme unique source d'azote des α-amino-carboxamides sous forme du racémate ou de ses isomères optiquement actifs, de formule générale 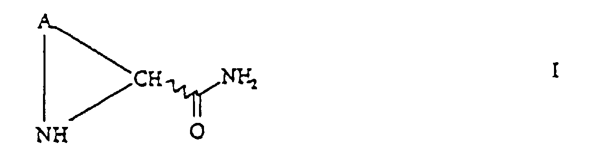 où A représente avec -NH et -CH un cycle hétérocyclique saturé à 5 ou 6 chaînons éventuellement substitué, et de convertir des (RS)-α-aminocarboxamides de formule I déjà définie en un acide S-α-aminocarboxylique de formule générale 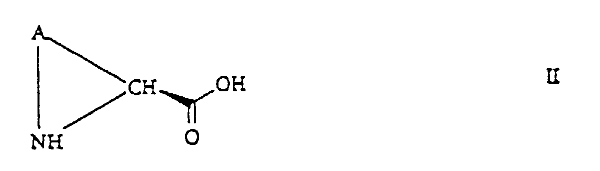 où A a la signification citée.

2. Microorganismes selon la revendication 1 qui sont capables d'utiliser comme unique source d'azote le pipécolinamide ou le pipérazinecarboxamide sous forme du racémate ou de ses isomères optiquement actifs.

3. Microorganismes selon la revendication 2 du genre Klebsiella ou Pseudomonas.

4. Microorganismes selon la revendication 3 de l'espèce Pseudomonas putida DSM 9923, de l'espèce Pseudomonas fluorescens DSM 9924, de l'espèce Klebsiella terrigena DSM 9174, de l'espèce Klebsiella pneumoniae DSM 9175 et DSM 9176, ainsi que leurs variants et mutants fonctionnellement équivalents.

5. Procédé de préparation d'acides S-α-aminocarboxyliques de formule générale 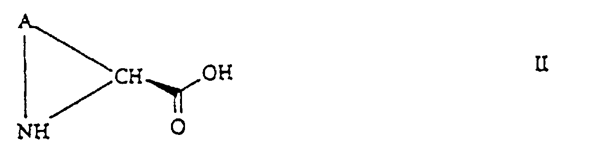 où A a la signification citée dans la revendication 1 et/ou de R-α-aminocarboxamides de formule générale 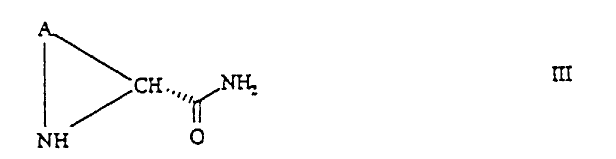 où A a la signification citée, caractérisé en ce que, dans le (RS)-α-aminocarboxamide de formule générale 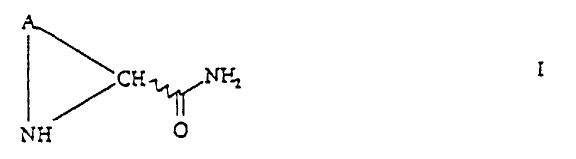 où A a la signification citée, le S-α-aminocarboxamide est converti en l'acide S-α-aminocarboxylique au moyen des microorganismes selon la revendication 1 ou au moyen d'enzymes sans cellules provenant de ces microorganismes et est isolé, où, lors de la biotransformation, outre l'acide S-α-aminocarboxylique, il se forme le R-α-aminocarboxamide qui est éventuellement isolé.

6. Procédé selon la revendication 5 caractérisé en ce que l'on opère la biotransformation au moyen de microorganismes de l'espèce Klebsiella terrigena DSM 9174, ou Klebsiella pneumoniae DSM 9175 et DSM 9176 ou avec leurs variants et mutants fonctionnellement équivalents ou avec des enzymes sans cellules provenant de ces microorganismes.

7. Procédé selon la revendication 5 ou 6 caractérisé en ce que l'on utilise comme (RS)-α-aminocarboxamide le (RS)-pipécolinamide. le (RS)-pipérazinecarboxamide, le (RS)-4-méthylpipécolinamide ou le (RS)-prolinamide.

8. Procédé selon au moins l'une des revendications 5 à 7 caractérisé en ce que l'on conduit la biotransformation par addition unique ou continue du (RS)-α-aminocarboxamide de sorte que la concentration du (RS)-α-aminocarboxamide dans le milieu de culture ne dépasse pas 20 % en masse.

9. Procédé selon au moins l'une des revendications 5 à 8 caractérisé en ce que l'on conduit la biotransformation à un pH de 7 à 10 et à une température de 30 à 60°C.
